Europäisches Patentamt

European Patent Office

Office européen des brevets

(19)

(11) Veröffentlichungsnummer : **0 014 431**
**B1**

(12) **EUROPÄISCHE PATENTSCHRIFT**

(45) Veröffentlichungstag der Patentschrift :
**11.08.82**

(21) Anmeldenummer : **80100470.6**

(22) Anmeldetag : **30.01.80**

(51) Int. Cl.³ : **B 01 J 49/00, C 07 C 53/21,
C 07 C 51/47, C 08 F 6/14,
C 08 F 14/18**

(54) **Verfahren zur Rückgewinnung fluorierter Emulgatorsäuren aus basischen Anionenaustauschern.**

(30) Priorität : **02.02.79 DE 2903981**

(43) Veröffentlichungstag der Anmeldung :
**20.08.80 (Patentblatt 80/17)**

(45) Bekanntmachung des Hinweises auf die Patenterteilung : **11.08.82 Patentblatt 82/32**

(84) Benannte Vertragsstaaten :
**DE FR GB IT NL**

(56) Entgegenhaltungen :
**DE A 1 085 878
DE A 2 044 986
US A 2 664 441**

(73) Patentinhaber : **HOECHST AKTIENGESELLSCHAFT
Postfach 80 03 20
D-6230 Frankfurt/Main 80 (DE)**

(72) Erfinder : **Kuhls, Jürgen, Dr.
Unghausen 16a
D-8263 Burghausen/Salzach (DE)**

Imprimerie Jouve, 18, rue St-Denis, 75001 Paris, France

# 0 014 431

## Verfahren zur Rückgewinnung fluorierter Emulgatorsäuren aus basischen Anionenaustauschern

Die Erfindung betrifft ein Verfahren zur Elution von an basischen Anionenaustauschern adsorbierten fluorierten Emulgatorsäuren.

Für die Polymerisation fluorierter Monomerer in wäßriger Dispersion können wegen der erforderlichen telogenen Inaktivität im allgemeinen nur fluorierte Emulgatoren mit Erfolg eingesetzt werden. Dies sind vor allem die Salze, vorzugsweise die Alkali- oder Ammoniumsalze von perfluorierten oder teilfluorierten Alkansäuren oder auch von perfluorierten oder teilfluorierten Alkansulfonsäuren. Die Herstellung solcher fluorierter Emulgatoren durch den Prozeß der Elektrofluorierung oder durch die Telomerisation fluorierter Monomerer ist mit hohen Kosten verbunden, die wiederum den hohen Preis solcher fluorierter Emulgatoren bedingen. Es ist daher von besonderer Bedeutung, zur Einsparung von Kosten den in der nach der Koagulation des Polymeren aus der Dispersion oder bei der Aufkonzentrierung wäßriger Polymerdispersionen in der wäßrigen Phase gelösten Anteil an fluoriertem Emulgator zurückzugewinnen. Die kann besonders vorteilhaft durch Adsorption mit Hilfe von Ionenaustauschern und deren anschließende Elution bewirkt werden.

In der DE-C 20 44 986 ist ein Verfahren zur Adsorption von Perfluorcarbonsäuren bzw. deren Salzen aus dem wäßrigen Serum gefällter Polytetrafluorethylen-Dispersionen mit Hilfe von schwach basischen Anionenaustauschern beschrieben. Nach diesem Verfahren soll die anschließende Elution des adsorbierten fluorierten Emulgators unter Verwendung einer verdünnten, wäßrigen Ammoniaklösung erfolgen.

Dieses Verfahren besitzt zwei wesentliche Nachteile. Erstens werden für die vollständige Elution relativ große Mengen an verdünnter NH$_4$OH-Lösung benötigt. Zum andern ist der Zeitbedarf zur Erreichung dieses Ziels zu hoch. Außerdem müssen die Ionenaustauscher nach beendetem Elutionsschritt in einem angeschlossenen Regenerierprozeß mit verdünnter Mineralsäure wieder in die für die erneute Adsorption der im allgemeinen in Salzform vorliegenden fluorierten Emulgatoren besonders geeignete Anionenform, insbesondere die Chloridform, übergeführt werden.

Der vorliegenden Erfindung lag daher die Aufgabe zugrunde, die für die Elution benötigte Menge an Elutionsmittel und damit auch den Zeitaufwand wesentlich zu reduzieren und ferner den Anionenaustauscher im gleichen Arbeitsgang für die erneute Adsorption der fluorierten Emulgatorsäuren zu regenerieren.

Diese Aufgabe wird gemäß der vorliegenden Erfindung dadurch gelöst, daß die Elution der adsorbierten fluorierten Emulgatorsäure aus dem Anionenaustauscher mit einem Gemisch aus verdünnter Mineralsäure und einem organischen Lösungsmittel vorgenommen wird.

Unter dem Begriff fluorierte Emulgatorsäuren sollen hier vor allem perfluorierte Alkansäuren der Formel $CF_3(CF_2)_nCOOH$ (n = 3 bis 10), insbesondere Perfluoroctansäure, verstanden werden, auf die das erfindungsgemäße Verfahren bevorzugt anwendbar ist. Unter diesen Begriff fallen aber auch teilfluorierte Alkansäuren der Formel $XCF_2(CF_2)_nCOOH$ (X = H oder Cl, n = 3 bis 10), perfluorierte oder teilfluorierte Alkansulfonsäuren der Formel $XCF_2(CF_2)_nSO_3H$ (X = H oder vorzugsweise F, n = 3 bis 10) sowie Perfluor-[(β-propoxy)-propionsäure]. Auch diese fluorierten Emulgatorsäuren können nach dem erfindungsgemäßen Verfahren eluiert werden. Es können auch Gemische der genannten fluorierten Emulgatorsäuren adsorbiert und eluiert werden, insbesondere solche, die Perfluoroctansäure als Hauptbestandteil enthalten.

Die genannten fluorierten Emulgatorsäuren werden dem Anionenaustauscher in den aufzuarbeitenden Lösungen üblicherweise in Form ihrer Salze, insbesondere ihrer Alkali- und Ammoniumsalze zur Adsorption aufgegeben. Diese aufzuarbeitenden Lösungen können daneben auch Fluoridionen, ebenso aber auch andere Dispergiermittel enthalten, wie z.B. nichtionische Oxalkylate von Alkoholen oder Phenolen, ohne daß dadurch die Rückgewinnung der fluorierten Emulgatorsäuren nach dem erfindungsgemäßen Verfahren behindert wird.

Als Mineralsäuren kommen alle diejenigen in Frage, deren Anionen dem Anionenaustauscher eine Salzform (Anionenform) verleihen, die für die weitere Adsorption von fluorierten Emulgatorsäuren geeignet ist. Ihre Oxidationsstärke unter den Bedingungen der Elution soll so gering sein, daß der Anionenaustauscher nicht oxidativ geschädigt wird. Geeignete Mineralsäuren sind beispielsweise Ortho-, Meta- oder Diphosphorsäure, Salpetersäure und vorzugsweise Salzsäure und Schwefelsäure.

Als organische Lösungsmittel kommen im Prinzip alle organischen Lösungsmittel in Betracht, die im verwendeten sauerwäßrigen System weder den eingesetzten Anionenaustauscher in seiner Funktion, beispielsweise durch Quellung oder Anlösen, schädigen noch mit der eingesetzten Mineralsäure unerwünschte Reaktionen eingehen. Derartige Lösungsmittel können polare organische Lösungsmittel sein, wie beispielsweise Chlorkohlenwasserstoffe, aliphatische oder aromatische Ether und Carbonsäureester. Bevorzugt sind jedoch mit Wasser weitgehend, das heißt bei Vermischung gleicher Volumina zu 40 bis 100 %, oder unbegrenzt mischbare Lösungsmittel, wie vorzugsweise aliphatische Alkohole mit 1 bis 4 C-Atomen, insbesondere Methanol oder Ethanol, cyclische Ether wie Tetrahydrofuran oder Dioxan, kurzkettige, dialkylsubstituierte Amide wie Dimethylformamid oder Dimethylacetamid oder auch Mono- oder Dimethyl- oder Mono- oder Diethylether des Ethylenglykols oder der entsprechenden Polyglykole bis zur Kettenlänge des Dekaethylenglykols. Es können auch Gemische solcher Lösungsmittel Verwendung finden.

2

Aus der jeweils eingesetzten Mineralsäure und dem organischen Lösungsmittel wird ein Gemisch eingestellt, dessen Säurestärke, bezogen auf das Gesamtgemisch, 0,5 bis 10 n, vorzugsweise 0,5 bis 2 n, ist. Das Verhältnis von Mineralsäure (einschließlich des Wasseranteils) zu organischem Lösungsmittel in diesem Gemisch beträgt 1 : 0,25 bis 1 : 20, vorzugsweise 1 : 3 bis 1 : 10 Volumenteile.

Für die der Elution vorangehende Adsorption der fluorierten Emulgatorsäuren werden vorzugsweise schwach basische Anionenaustauscher eingesetzt, die primäre, sekundäre, vorzugsweise aber tertiäre Aminogruppen in Form der Ammoniumsalze enthalten (beispielsweise die Typen MP 62 der Firma Bayer AG, Leverkusen, oder IRA 68 der Firma Serva, Heidelberg). Geeignet sind im Prinzip auch stark basische Anionenaustauscher, die in der Regel quaternäre Ammoniumgruppen besitzen (beispielsweise Typ M 600 der Firma Bayer AG, Leverkusen). Diese stark basischen Anionenaustauscher besitzen für die zu adsorbierenden Emulgatorsäuren bzw. deren Salze jedoch im allgemeinen nur in der OH-Form ein ausreichendes Adsorptionsvermögen und müssen daher nach beendeter Elution durch Zugabe von starken Basen, wie beispielsweise Natronlauge, wieder in die OH-Form übergeführt werden.

Für die vollständige Elution der adsorbierten Emulgatorsäuren wird im allgemeinen eine Menge von 50 bis 500, vorzugsweise 100 bis 225 Vol.-Teile, bezogen auf 100 Vol.-Teile an Füllung des Anionenaustauschers, der Mischung aus Mineralsäure und organischem Lösungsmittel benötigt.

Nach beendeter Elution trennt sich das Eluat in der Regel in zwei Schichten, wobei die spezifisch schwerere, untere Schicht praktisch die gesamte fluorierte Emulgatorsäure enthält. Diese wird mit verdünnter, üblicherweise etwa 2 n Natronlauge neutralisiert und dann durch Einrühren in verdünnte Salzsäure in kompakter und leicht abtrennbarer Form ausgefällt.

Die Temperatur bei der Elution nach dem erfindungsgemäßen Verfahren ist nicht kritisch. Die Elution soll zweckmäßigerweise bei Raumtemperatur vorgenommen werden, also etwa im Temperaturbereich von 15 bis 25 °C, gelegentlich kann eine Temperaturerhöhung von Vorteil sein.

Das erfindungsgemäße Verfahren besitzt gegenüber der bekannten Elution mit verdünnter, wäßriger Ammoniaklösung wesentliche Vorteile. Wie aus Tabelle I ersichtlich, kann die benötigte Menge an Elutionsmittel drastisch reduziert und damit der zeitbedarf bis zur Entladung des Anionenaustauschers verkürzt werden. Dabei wird die Ausbeute an zurückgewonnener fluorierter Emulgatorsäure erhöht. Sie ist in vielen Fällen nahezu quantitativ. Durch die erfindungsgemäße Verwendung von verdünnten anorganischen Mineralsäuren im Gemisch mit organischen Lösungsmitteln wird während der Elution gleichzeitig im Falle des Einsatzes eines schwach basischen Anionenaustauschers dieser wieder in seine ursprüngliche Salzform übergeführt. Damit wird die sonst erforderliche separate Regenerierung eines solchen schwach basischen Anionenaustauschers in die Salzform mittels verdünnter Mineralsäure nach jedem Elutionsschritt eingespart.

Die Abtrennung und Wiedergewinnung von fluorierten Emulgatorsäuren hat Bedeutung vor allem bei wäßrigen Lösungen, die bei Fällungs- und Koagulationsvorgängen im Zuge der Aufarbeitung von Emulsionspolymerisaten fluorhaltiger Monomerer, also vor allem Homo- und Mischpolymerisaten des Tetrafluorethylens, Vinylidenfluorids, Vinylfluorids und Trifluorchlorethylens, aber auch von in wäßriger Emulsion gewonnenen Fluorcarbon-Telomerwachsen anfallen. Desgleichen kann die Abtrennung und Wiedergewinnung fluorierter Emulgatorsäuren auch aus wäßrigen Lösungen vorgenommen werden, die bei Aufkonzentrierungsprozessen von Fluorpolymerisat-Latices gewonnen werden, insbesondere bei solchen Aufkonzentrierungsverfahren, die unter Schichtentrennung verlaufen. Soweit in solchen wäßrigen Lösungen noch Reste von Katalysatoren, anorganische Salze oder nichtionische Dispergiermittel, wie beispielsweise alkoxylierte Alkylphenole oder alkoxylierte aliphatische Alkohole oder Alkylenoxid-Blockpolymerisate, enthalten sind, stören sie die Adsorption und auch die Elution nicht.

Tabelle I

Verfahrensvergleich der Elution von Perfluoroctansäure mit Ammoniak oder mit Gemischen von verdünnter Mineralsäure und organischem Lösungsmittel

| Verfahrensschritt | Beispiel 1<br>DE-C 20 44 986 | Erfindungsgemäßes Verf.<br>Beispiel 4, Tabelle II |
|---|---|---|
| 1. Elution | | |
| Elutions-Mittel | 1 n NH$_4$OH | HCl konz./Ethanol |
| Menge (cm$^3$) | 800 | 50/250 |
| Elutions-Zeit (min) | 60 | 10 |
| 2. Regenerierung des Ionenaustauschers | Behandlung mit 800 cm$^3$ 1 n HCl | nicht erforderlich |

In beiden Fällen wurde die Adsorption der Perfluoroctansäure mit Hilfe der durch Behandlung mit verdünnter Salzsäure hergestellten Chloridform eines schwach basischen Anionenaustauschers (MP 62, Hersteller Bayer, Leverkusen) durchgeführt. Abmessungen und Füllungsgrad der Austauschersäule sind in der folgenden allgemeinen Arbeitsvorschrift angegeben.

# 0 014 431

Die nachfolgenden Beispiele sollen die vorliegende Erfindung näher erläutern, ohne diese damit in ihrem Umfang einzuschränken.

## Arbeitsvorschrift

### a) Adsorption

Falls nicht anders vermerkt, wurden 200 cm³ des schwach basischen Anionenaustauschers MP 62 (Hersteller : Bayer AG, Leverkusen) in eine, mit Glasfritte versehene, zylindrische Glassäule (Länge : 64 cm ; Durchmesser : 20 mm) gegeben und durch Spülung mit 800 cm³ 1 n Mineralsäure in die in Tabelle II, Spalte 2, angegebene Salzform übergeführt. Anschließend wurden 2 l einer 70 g Perfluoroctansäure enthaltenden, mit verdünntem Ammoniak auf pH 4 eingestellten wäßrigen, aus der Polytetrafluorethylen-Polymeraufarbeitung stammenden Lösung innerhalb einer Stunde über den Austauscher geleitet, wobei die in der Tabelle II, Spalte 3, angegebene Menge auf der Säule adsorbiert wurde.

### b) Elution

In Tabelle II, Spalte 4, finden sich Angaben über Zusammensetzung und Menge des Elutionsgemisches sowie den Zeitbedarf bis zur Erreichung des angegebenen Perfluoroctansäure-Elutionsgrads. Das Eluat bestand in der Regel aus zwei Schichten, von denen die spezifisch schwerere untere Schicht in allen Fällen nahezu die gesamte Perfluoroctansäure enthielt. Diese wurde von der überstehenden Schicht abgetrennt, mit 2 n NaOH neutralisiert und durch Einrühren in 1 000 cm³ 2 n Salzsäure zur Fällung gebracht. Die ausgefallenen Perfluoroctansäurekristalle wurden anschließend filtriert, mit verdünnter Salzsäure gewaschen und getrocknet.

## Tabelle II

Adsorption und Rückgewinnung von Perfluoroctansäure aus basischen Anionenaustauschern

| Beispiel Nr. | Salzform des Austauschers | Adsorption der Perfluoroctansäure g | Elution der Perfluoroctansäure mit organ. Lösungsmittel (cm³) | | Mineralsäure (1) (cm³) | | Zeitbedarf min | Eluiert g | % v. adsorb. Menge |
|---|---|---|---|---|---|---|---|---|---|
| 1 | Cl⁻ | 67 | Methanol | (250) | HCl konz. | (50) | 10 | 65 | 97 |
| 2 | OH⁻ | 10 | Methanol | (250) | HCl konz. | (50) | 12 | 8 | 80 |
| 3 | $SO_4^{2-}$ | 60 | Methanol | (250) | HCl konz. | (50) | 11 | 45 | 75 |
| 4 | Cl⁻ | 62 | Ethanol | (250) | HCl konz. | (50) | 10 | 60 | 97 |
| 5 | Cl⁻ | 68 | n-Propanol | (250) | HCl konz. | (50) | 12 | 67 | 99 |
| 6 | Cl⁻ | 60 | Dioxan | (250) | HCl konz. | (50) | 11 | 60 | 100 |
| 7 | Cl⁻ | 62 | Diethylether | (250) | HCl konz. | (50) | 13 | 40 | 65 |
| 8 | Cl⁻ | 64 | Dimethylformamid | (250) | HCl konz. | (50) | 10 | 61 | 95 |
| 9 | Cl⁻ | 64 | Methylenchlorid | (250) | HCl konz. | (50) | 14 | 32 | 50 |
| 10 | Cl⁻ | 60 | Methanol | (250) | $H_2SO_4$ konz. | (25) | 10 | 50 | 83 |
| 11 | OH⁻ (2) | 52 | Methanol | (250) | HCl konz. | (50) | 12 | 38 | 73 |
| 12 | OH⁻ (2) | 50 | Methanol | (250) | $H_2SO_4$ konz. | (25) | 12 | 35 | 70 |
| 13 | Cl⁻ | 60 | Methanol | (500) | HCl konz. | (100) | 12 | 50 | 83 |
| 14 | OH⁻ (3) | 68 | Methanol | (250) | HCl konz. | (50) | 13 | 50 | 83 |
| 15 | Cl⁻ | 59 (4) | Ethanol | (250) | HCl konz. | (50) | 15 | 39 | 66 |
| 16 | Cl⁻ | 67 | Ethanol | (250) | $H_3PO_4$ (50 g) + $H_2O$ | (150) | 20 | 32 | 48 |

(1) HCl konz. = 37 Gew.-%, $H_2SO_4$ konz. = 98 Gew.-%, $H_3PO_4$ = 85 Gew.-%.
(2) Stark basischer Anionenaustauscher M 600 (Bayer AG, Leverkusen).
(3) Schwach basischer Anionenaustauscher IRA-68, Amberlite (Fa. Serva, Heidelberg).
(4) Es wurde Perfluor-n-octansulfonsäure n-$C_8F_{17}SO_3H$ eingesetzt ; Gehalt vor der Aufgabe auf den Anionenaustauscher 70 g pro 2 l.

## Ansprüche

1. Verfahren zur Elution von an basischen Anionenaustauschern adsorbierten fluorierten Emulgatorsäuren, dadurch gekennzeichnet, daß die Elution der adsorbierten fluorierten Emulgatorsäure aus dem Anionenaustauscher mit einem Gemisch aus verdünnter Mineralsäure und einem organischen Lösungsmittel vorgenommen wird.

2. Verfahren nach Anspruch 1, dadurch gekennzeichnet, daß eine 0,5 bis 10 n Mineralsäure im

4

Gemisch mit organischem Lösungsmittel eingesetzt wird, wobei das Verhältnis wäßrige Mineralsäure zu organischem Lösungsmittel 1 : 0,25 bis 1 : 20 beträgt.

3. Verfahren nach Anspruch 1 und 2, dadurch gekennzeichnet, daß die zu eluierende fluorierte Emulgatorsäure eine Perfluorcarbonsäure der Formel $CF_3 (CF_2)_n COOH$, worin n = 3 bis 10 bedeutet, ist.

4. Verfahren nach Anspruch 1 bis 3, dadurch gekennzeichnet, daß als verdünnte Mineralsäure Salzsäure verwendet wird.

5. Verfahren nach Anspruch 1 bis 4, dadurch gekennzeichnet, daß ein organisches Lösungsmittel eingesetzt wird, das zu mindestens 40 Vol.-% mit Wasser mischbar ist.

## Claims

1. Process for eluting fluorinated emulsifier acids adsorbed on basic anion exchangers, which is characterised by eluting the adsorbed fluorinated emulsifier acid from the anion exchanger with a mixture of a dilute mineral acid and an organic solvent.

2. The process of claim 1, which is characterised in that 0.5 to 10 N mineral acid is used in admixture with an organic solvent and the proportion of aqueous mineral acid to organic solvent is in the range of from 1 : 0.25 to 1 : 20.

3. The process of claims 1 and 2, which is characterised in that the fluorinated emulsifier acid to be eluted is a perfluorocarboxylic acid of the formula $CF_3(CF_2)_n COOH$ in which n is a number from 3 to 10.

4. The process of claims 1 to 3, which is characterised in that hydrochloric acid is used as dilute mineral acid.

5. The process of claims 1 to 4, which is characterised in that the organic solvent is miscible with water to an extent of at least 40 % by volume.

## Revendications

1. Procédé pour éluer des acides fluorés émulsionnants qui ont été adsorbés sur des échangeurs d'anions basiques, procédé caractérisé en ce qu'on effectue l'élution de l'acide fluoré émulsionnant adsorbé hors de l'échangeur d'anions à l'aide d'un mélange constitué d'un acide minéral dilué et d'un solvant organique.

2. Procédé selon la revendication 1, caractérisé en ce qu'on utilise un acide minéral de normalité comprise entre 0,5 et 10 en mélange avec un solvant organique, le rapport de l'acide minéral aqueux au solvant organique étant compris entre 1 : 0,25 et 1 : 20.

3. Procédé selon l'une des revendications 1 et 2, caractérisé en ce que l'acide fluoré émulsionnant à éluer est un acide perfluorocarboxylique répondant à la formule $CF_3 (CF_2)_n\text{-}COOH$ dans laquelle n est un nombre de 3 à 10.

4. Procédé selon l'une quelconque des revendications 1 à 3, caractérisé en ce qu'on utilise l'acide chlorhydrique comme acide minéral dilué.

5. Procédé selon l'une quelconque des revendications 1 à 4, caractérisé en ce qu'on utilise un solvant organique qui est miscible à l'eau à au moins 40 % en volume.